# EUROPEAN PATENT APPLICATION

(11) **EP 1 935 253 A1**
(43) Date of publication of application: **25.06.2008**
(21) Application number: 06782089.4
(22) Date of filing: 01.08.2006
(51) Int. Cl.: A23K 1/16, A23K 1/165, A61K 36/06, A61P 31/04, A61P 33/02

(54) **ADDITIVE FOR ANIMAL FEED**

(30) Priority: 20.09.2005 JP 2005272049; 13.02.2006 JP 2006034953; 20.02.2006 JP 2006042138; 17.04.2006 JP 2006113092; 11.05.2006 JP 2006132793; 14.07.2006 JP 2006193915; 14.07.2006 JP 2006193920
(71) Applicant: Idemitsu Kosan Co., Ltd., Chiyoda-ku Tokyo 100-8321 (JP)
(72) Inventor: KADOTA, Akihiko, Sodegaura-shi Chiba 299-0205 (JP); SUZUKI, Motoshi, deceased (JP); ITO, Shinji, Sodegaura-shi Chiba 299-0293 (JP); SUGIMOTO, Yasuaki, Tokyo 130-0015 (JP); MOCHIZUKI, Masami, Sodegaura-shi Chiba 299-0293 (JP)
(74) Representative: Vossius & Partner
(86) International application number: PCT/JP2006/315211
(87) International publication number: WO 2007/034627

(57) **Abstract**

To promote the digestion of an animal to thereby elevate the feed efficiency or prevent or ameliorate intestinal infections such as inflammatory intestinal injury of the animal, a combination of at least one fungus selected from *Aspergillus sojae, Aspergillus tamarii, Aspergillus foetidus, Aspergillus niger,* and *Aspergillus oryzae* with an acidic enzyme produced by the fungus is administered to the animal.

## Description

### Technical Field

The present invention relates to additives for animal feed containing an *Aspergillus* fungus having an ability to produce an acidic enzyme.

### Background Art

Feeds for livestock or pet animals (hereinafter, referred to as animals) are manufactured through a process such as pulverization, but are generally not subjected to heat treatment or the like. Accordingly, the feeds have problems of a low digestion and absorption rate and a low feed efficiency. A recent report revealed that inflammatory intestinal injury such as ulcerative colitis or Crohn's disease, which is known as a human disease, may occur in animals and cause a diarrhea symptom or the like. In addition, such inflammatory intestinal injury described above is known to decrease absorption of a feed and prevent healthy fattening. Examples of a pathogen that causes intestinal infections of an animal include pathogenic *Escherichia coli, Salmonella, Clostridium,* and *Campylobacter.* Abnormal proliferation of such pathogens may produce toxins (enterotoxin, cytotoxin) to cause disorders in the intestinal mucosa, resulting in loose stool, severe diarrhea, etc. Meanwhile, the coccidium is a protozoa that is parasitic in the intestines of chickens, pigs, cows, etc., and infection of coccidium may cause diarrhea, anorexia, or the like. Antibiotics have been used to prevent/treat such inflammatory intestinal injury, but the antibiotics may lead to the appearance of drug-resistant bacteria.

In recent years, technologies for improving the balance of enterobacterial flora and suppressing proliferation of pathogens in the intestine using probiotics have attracted attentions (Patent Documents 1 and 2). However, many of lactic acid bacteria and bifidobacteria may die under a condition of 0.3% deoxycholic acid or pH 4 or lower, and many of bacterial strains other than bacteria collectively referred to as coliform group cannot be present in the presence of deoxycholic acid, which is one of bile acids and has a strong antibacterial activity against microorganisms. Therefore, a bacterial strain that does not die in the digestive tract of an animal and provides an effect favorable to a host has been desired.

On the other hand, it has been reported that a technology for reducing the burden of the stomach and intestines of animals by treating an animal feed with an enzyme or an enzyme-producing fungus in a processing step of the feed to degrade the feed to some degree is useful for prevention or amelioration of gastrointestinal diseases (Patent Document 3). In addition, there is known a method of producing a fermented fish meal feed for fish, which contains a protease, a lipase, etc. at a high level by fermenting fish meal using Koji mold at a low moisture content (Patent Document 4). However, such technologies have a problem in that cumbersome steps of increasing the moisture content in the feed to degrade components of the feed and drying the resultant product for commercialization are necessary. Moreover, in the feed having a moisture content increased for the purpose of degradation of the components of the feed, putrefying bacteria or fungi easily appear, resulting in difficulty in quality maintenance.
In addition, a method of modifying feces of animals by orally administering spores of Koji mold to the animals has been reported, but no study has been made on the intended purposes such as suppression of proliferation of bacterium that causes inflammation in the intestine of an animal and promotion of weight gain, and the fact that Koj i mold is allowed to produce an acidic enzyme and administered to an animal is not disclosed (Patent Document 5).
Moreover, a method including mixing a pulverized product of the shell of a crustacean with a fermentation nutrient added thereto, inoculating an *Aspergillus* fungus thereinto, degrading chitin and chitosan by the fungus, and administering the resultant to animals is described (Patent Document 6). The growth-promoting effect of the method has been partially clarified. However, no study has been made on suppression of proliferation of a pathogen in the intestine of an animal to prevent/treat intestinal infections, and the effect has not been proved.

On the other hand, *Aspergillus sojae, Aspergillus tamarii,* and *Aspergillus oryzae* are known to produce Kojic acid (Non-patent Document 1). Meanwhile, it has been reported that Kojic acid has antibacterial activities against *Aerobacter, Alcaligenes, Bacillus, Brucella, Chromobacterium, Clostridium, Corynebacterium, Diplococcus, Eberthella, Escherichia, Klebsiella, Leptospira, Micrococcus, Neisseria, Pasteurella, Proteus, Pseudomonas, Salmonella, Sarcina, Shigella, Serratia, Spirillum, Staphylococcus, Streptococcus,* and *Vibrio* (Non-patent Document 2). However, formation of liver cell tumor was observed in mice administered with Kojic acid, and Kojic acid is suspected to be cancer-causing in the livers of rats. In addition, whether Kojic acid has genetic toxicityornot is unclear, but the possibility of the genetic toxicity cannot be denied. It must be careful to determine to add the Koji acid in the feed.

Patent Document 1: JP 2005-507670 A
Patent Document 2: JP 2004-523241 A
Patent Document 3: JP 2004-141147 A
Patent Document 4: JP 06-319464 A
Patent Document 5: JP 11-171674 A
Patent Document 6: JP 2002-238466 A
Non-patent Document 1: George A, Burdock, Madhusudan G. Soni, and Iona G. Carabin (2001) Regulatory Toxicology and Pharmacology 33, 80-101
Non-patent Document 2: Harry E. Morton, Walter Kocholaty, Renate Junowicz-Kocholaty, and Albert Kelner (1945) J. Bacteriol 50, 579-584

### Disclosure of the Invention

An object of the present invention is to provide a safe and simple means for promoting the digestion of an animal in order to elevate the feed efficiency. Specifically, an object of the present invention is to provide means for preventing/treating an infection by suppressing proliferation of a pathogen or coccidium in the intestine of an animal to achieve weight gain of the animal.

The inventors of the present invention have made extensive studies to achieve the above-mentioned objects. As a result, the inventors have discovered that: *Aspergillus sojae, Aspergillus tamarii, Aspergillus foetidus, Aspergillus niger,* and *Aspergillus oryzae* have excellent abilities to produce acidic enzymes, in particular, acid amylase; those fungi have antibacterial activities against a pathogen that causes intestinal infections and protozoa-killing activities against coccidium; and those fungi can act as probiotics. The inventors have further discovered that the abilities of those fungi to produce acidic enzymes are extremely excellent in the case where those fungi are cultured using brown rice as a nutrient. Moreover, the inventors have found out that administration of the fungus bodies and acidic enzymes produced by the fungus bodies together with a feed to an animal can promote digestion of the animal, prevent/ameliorate intestinal infections, and contribute to weight gain of the animal, thereby completing the present invention.

That is, the present invention provides:
(1) an additive for animal feed comprising a culture containing at least one fungus selected from *Aspergillus sojae, Aspergillus tamarii, Aspergillus foetidus, Aspergillus niger,* and *Aspergillus oryzae,* and an acidic enzyme produced by the fungus;
(2) the additive for animal feed according to Item (1), wherein the fungus is *Aspergillus sojae* and/or *Aspergillus oryzae*;
(3) the additive for animal feed according to Item (1) or (2), wherein the *Aspergillus oryzae* is *Aspergillus oryzae* IK-05074 strain (FERM BP-10622) or a mutant of the *Aspergillus oryzae* IK-05074 strain having the same ability to produce acidic enzyme as the *Aspergillus oryzae* IK-05074 strain;
(4) the additive for animal feed according to any one of Items (1) to (3), wherein the acidic enzyme is an acid amylase;
(5) the additive for animal feed according to any one of Items (1) to (4), wherein the fungus has antibacterial activity against a pathogen that causes an intestinal infection of an animal and/or protozoa-killing activity against a coccidium;
(6) the additive for animal feed according to any one of Items (1) to (5), wherein the culture contains a plant-derived nutrient;
(7) the additive for animal feed according to Item (6), wherein the plant-derived nutrient is brown rice;
(8) a feed comprising the additive for animal feed according to any one of Items (1) to (7);
(9) a method of manufacturing a feed comprising: culturing at least one fungus selected from *Aspergillus sojae, Aspergillus tamarii, Aspergillus foetidus, Aspergillus niger,* and *Aspergillus oryzae* on a solid medium containing a nutrient for proliferation of the fungus; and adding the resultant culture to a feed.

### Best Mode for carrying out the Invention

*Aspergillus sojae, Aspergillus tamarii, Aspergillus foetidus, Aspergillus niger,* or *Aspergillus oryzae* comprised in an additive for animal feed of the present invention is fungus classified into each of the above-mentioned species by a method that is generally used in the art for identification of the species of *Aspergillus* fungi. The identification of the species of the fungi can be performed with reference to, for example, "H. Murakami, The JournalofGeneraland Applied Microbiology, 17, p.281-309, (1971)," "Hideya Murakami, Journal of the Brewing Society of Japan, 74 (12), p.849-853, (1979)," "Nikkuni, S., et al, The Journal of General and Applied Microbiology, 44, p.225-230, (1998)," etc.

*Aspergillus sojae* is one of filamentous imperfect fungi present in soil, Koji, etc. and is used for brewing of soy sauce or miso. In the additive for animal feed of the present invention, the fungus is not particularly limited as long as it has an ability to produce at least one of the acidic enzymes described in detail below, preferably an acid amylase, and is safe for animals. In the additive for animal feed of the present invention, the fungus may be a commercially available product, and preferable examples thereof include *Aspergillus sojae* AOK 210 strain (Akita Konno Co., Ltd.).

*Aspergillus tamarii* is one of filamentous imperfect fungi present in soil, Koji, foods, etc. and is used for brewing of soy sauce or miso. In the additive for animal feed of the present invention, the fungus is not particularly limited as long as it has an ability to produce at least one of the acidic enzymes described in detail below, preferably an acid amylase, and is safe for animals. In the additive for animal feed of the present invention, the fungus may be a commercially available product, and preferable examples thereof include *Aspergillus tamarii* AOK 43 strain (Akita Konno Co. , Ltd.).

*Aspergillus foetidus* is one of filamentous imperfect fungi present in soil, Koji, grain residue, etc. and is used for sake, miso, or soy source. In the additive for animal feed of the present invention, the fungus is not particularly limited as long as it has an ability to produce at least one of the acidic enzymes described in detail below, preferably an acid amylase, and is safe for animals. In an additive for animal feed of the present invention, the fungus may be a commercially available product, and preferable examples thereof include *Aspergillus foetidus* AOK N4586 strain (Akita Konno Co., Ltd.).

*Aspergillus niger* is one of filamentous imperfect fungi present in soil, Koji, grain residue, etc. and is used for various fields such as a production of alcoholic beverages, food processing, production of sugar, and production of drugs. The fungus is not particularly limited as long as it has an ability to produce at least one of the acidic enzymes described in detail below, preferably an acid amylase, and is safe for animals. In an additive for animal feed of the present invention, the fungus may be a commercially available product, and preferable examples thereof include *Aspergillus niger* AOK B650 strain (Akita Konno Co., Ltd.).

Meanwhile, mutants of AOK 210 strain, AOK 43 strain, AOK N4586 strain, or AOK B650 strain may be used. The mutants can be obtained by selecting the strains having the same ability to produce acidic enzyme as AOK 210 strain, AOK 43 strain, AOK N4586 strain, or AOK B650 respectively from the strains obtained by natural mutation or by mutation treatment with a chemical mutagen or ultraviolet ray. Moreover, it is preferable to use mutants of the above-mentioned strains each having not only the same ability to produce an acidic enzyme but also at least one of the same antibacterial activity, protozoa-killing activity, bile acid resistance, and acid resistance as the above-mentioned strains. In addition, it is also preferable to use mutants each having mycological properties other than the above-mentioned properties, which are the same as those of AOK 210 strain, AOK 43 strain, AOK N4586 strain, or AOK B650.

*Aspergillus oryzae* is one of filamentous imperfect fungi present in soil, Koji, etc. and is used for brewing of soy sauce or miso. In the additive for animal feed of the present invention, the fungus is not particularly limited as long as it has an ability to produce at least one of the acidic enzymes described in detail below, preferably acid amylase, and is safe for animals. In an additive for animal feed of the present invention, the fungus may be a commercially available product, and preferable examples thereof include *Aspergillus oryzae* IK-05074 strain. IK-05074 strain was isolated from various fermented foods and was deposited on Feb. 15, 2006 at the International Patent Organism Depositary, National Institute of Advanced Industrial Science and Technology (Tsukuba Central 6, 1-1, Higashi 1-Chome, Tsukuba-shi, Ibaraki-ken, Japan) and given an accession number of FERM P-20798, and the deposit was then converted to an international deposit under the provisions of Budapest Treaty on Jun. 20, 2006 and given an accession number of FERM BP-10622.

The mycological properties of IK-05074 strain are as follows.
(1) Colony habit: Colony on Czapek-Dox-agar growing to a diameter of 50-60mmin 7 days at 25°C, yellow-green, turning brownish in age. Mycelium inconspicuous; reverse uncoloured.
(2) Morphology: Conidiophores thin to thick-walled, smooth walled to slightly roughend, up to 20µm ϕ and 2 mm long, terminating in globose vesicles of mostly 40-50 (-80) µm ϕ. Metulae mostly present in larger conidiophores, up to 12 µm long; phialides ampulliform, 8-12µm long, with a short neck; conidia more or less globose, mostly 5-6 µmϕ, yellow-green, surface smooth to finely roughened.

From the above-mentioned characteristics, IK-05074 strain was identified to belong to *Aspergillus oryzae.*

Meanwhile, a mutant of IK-05074 strain can be used in the additive for animal feed of the present invention. The mutant of IK-05074 strain can be obtained by selecting a strain having the same ability to produce acidic enzyme as IK-05074 strain from strains obtained by natural mutation or by mutation treatment with a chemical mutagen or ultraviolet ray. Moreover, it is preferable to use a mutants of IK-05074 strain having not only the same ability to produce an acidic enzyme but also at least one of the same antibacterial activity, protozoa-killing activity, bile acid resistance, and acid resistance as IK-05074 strain. In addition, it is also preferable to use a mutant having the same mycological properties as IK-05074 strain other than the above-mentioned properties.

Meanwhile, the additive for animal feed of the present invention may comprise an isolated strain having the ability to produce an acidic enzyme, which is selected from *Aspergillus sojae, Aspergillus tamarii, Aspergillus foetidus, Aspergillus niger,* and *Aspergillus oryzae* isolated from, for example, soil, Koji, foods, and grain residue.

The ability to produce an acidic enzyme is an ability to produce an acidic enzyme at a level enough to detect the activity of the acidic enzyme in a culture obtained by culturing a fungus body. The activity of an acidic enzyme in a culture can be detected in accordance with a conventional method. For example, the activity can be determined according to the glucoamylase activity measurement method, α-amylase activity measurement method, acid-resistant α-amylase activity measurement method, acid protease activity measurement method, and acid carboxypeptidase activity measurement method of the solid Koji analysis method defined in the National Tax Agency Japan prescribed analysis method (National Tax Agency first instruction, third revision).

*Aspergillus sojae, Aspergillus tamarii, Aspergillus foetidus, Aspergillus niger,* and *Aspelgillus oryzae,* which are used in the present invention, preferably have an antibacterial activity to pathogens causing intestinal infections in animals.
The above pathogens usually belong to *Enterobacteriaceae.* Examples of the pathogen belonging to a gram-negative bacterium include bacteria belonging to pathogenic *Escherichia coli, Salmonella* and *Campylobacter.* Examples of the pathogen belonging to a gram-positive bacterium include bacteria belonging to *Clostridium, Bacillus, Listeria, Staphylococcus* and *Streptococcus.*
Examples of the pathogenic *Escherichia coli* include *Enteroinvasive E. coli* (EIEC), *Enterotoxigenic E. coli* (ETEC), *Enteropathogenic E. coli* (EPEC), Shiga toxin producing *E. coli* (STEC) and *Enteroaggregative E. coli* (EAEC). Examples of the bacterium belonging to *Salmonella* include *S. pullorum, S. gallinarum, S. typhi, S. typhimurium, S. enteritidis, S. choleraesuis, S. derby and S. dublin*. Examples of the bacterium belonging to *Campylobacter* include *C. jejuni, C. coli, C. fetus,* and *C. fetus subsp. intestinalis.*
Examples of the bacterium belonging to *Clostridium* include *C. perfringens, C. botulinum and C. difficile.* An example of the bacterium belonging to *Bacillus* include *B. cereus*. Examples of the bacterium belonging to Listeria include *L. monocytogenes*. Examples of the bacterium belonging to *Staphylococcus* include *S*. *aureus.* Examples of the bacterium belonging to *Streptococcus* include *S. suis* and *S. pyogenes.* The above-mentioned fungi belonging to *Aspergillus* comprised in the additive for animal feed of the present invention have a high antibacterial activity particularly to bacteria belonging to *Salmonella,* more particularly to *S. enteritidis* and bacteria belonging to *Clostridium,* still more particularly to *C. perfringens, Escherichia coli* such as a bacterium causing edema disease, and *S. aureus.*

A fungus having the antibacterial activity against a pathogen means a fungus having the ability to suppress proliferation of a pathogen when the fungus is inoculated to a medium together with the pathogen. *Aspergillus sojae, Aspergillus tamarii, Aspergillus foetidus, Aspergillus niger,* and *Aspergillus oryzae* having antibacterial activities can be obtained by: adding an source for isolation such as soil or Koji to an agar medium containing a pathogen such as *Salmonella enteritidis* (SE), *Clostridium perfringens* (CP), *Escherichia coli* (EC), or *Staphylococcus aureus* (SA); culturing; and isolating fungus bodies that form inhibitory zones. The resultant fungi have the ability to suppress proliferation of the above-mentioned pathogens, and administration of the fungi to animals can prevent/treat intestinal infections of the animals.
Moreover, suppression of the proliferation of a pathogen that causes an intestinal infection of an animal can be confirmed by, for example, determining the concentration of pathogens (number of viable cells) in a cecal content or feces of the animal.

In addition, *Aspergillus sojae, Aspergillus tamarii, Aspergillus foetidus, Aspergillus niger,* and *Aspergillus oryzae* to be used in the present invention preferably have protozoa-killing activities against a coccidium that causes an intestinal infection of an animal.
The coccidium includes a protozoa classified into Sporozoa (subclass Sporozoasida). Specific examples thereof include the genera *Eimeria*, *Isospora, Taxoplasma,* and *Cryptosporidium.* Examples of a protozoa belonging to the genus *Eimeria* include *E. tenella, E. necatrix, E. acervulina, E. maxima, E. mitis, E. zuernii,* and *E. bovis.* Examples of a protozoa belonging to the genus *Isospora* include *I. suis, I. belli,* and *I. hominis.* Examples of a protozoa belonging to the genus *Toxoplasma* include *T. gondii.* Examples of a protozoa belonging to the genus *Cryptosporidium* include *C. parvum.* The additive for animal feed of the present invention can be particularly suitably used for infections caused by *E. tenella* and *E. zuernii.*

A fungus having the protozoa-killing activity against a coccidium means a fungus having the ability to suppress germination/proliferation of oocysts of coccidium, preferably to reduce the oocysts when the oocysts are present together with a culture of a fungus. Specifically, the fungus is one having the ability to deform/dissolve cell walls of oocysts to degrade the oocysts. The degradation of the oocysts and conditions of cell can be observed under a microscope.
*Aspergillus sojae, Aspergillus tamarii, Aspergillus foetidus, Aspergillus niger,* and *Aspergillus oryzae* having the protozoa-killing activities against a coccidium can be obtained by the following method, for example. An isolation source such as soil or Koji is added to a dish containing a suspension of oocysts of *E. tenella* or *E. zuernii* prepared with sterilized water, and the suspension is cultured at 37°C and observed for 1 to 7 days. Fungus bodies are isolated from the dish where oocysts are deformed or dissolved, and the fungus bodies are added to a dish containing a suspension of oocysts of *E. tenella* or *E. zuernii* prepared with sterilized water again and cultured at 37°C, followed by observation of deformed or dissolved oocysts. An *Aspergillus* fungus to be used in the present invention can be obtained by: culturing a fungus contained in a dish as described above; and selecting a strain having the mycological properties of *Aspergillus sojae, Aspergillus tamarii, Aspergillus foetidus, Aspergillus niger,* or *Aspergillus oryzae.* Administration of a fungus obtained as described above to animals can prevent/treat intestinal coccidium infections of the animals.

Meanwhile, suppression of proliferation of coccidium by administration of the above-mentioned *Aspergillus* fungi can be confirmed by, for example, observing oocysts of coccidium in cecal contents or feces of animals under a microscope.

*Aspergillus sojae, Aspergillus tamarii, Aspergillus foetidus, Aspergillus niger,* or *Aspergillus oryzae* comprised in the additive for animal feed of the present invention preferably has resistance to gastric acid or bile acid. The resistance may enable production of a useful acidic enzyme by fungus bodies in the intestine of an animal and maintenance of digestion-facilitating function of the acidic enzyme. In addition, in the case where the above-mentioned *Aspergillus* fungus have antibacterial activities against pathogens as described above, proliferation of a pathogen that causes an intestinal infection of an animal can be suppressed and the balance of enteric flora can be improved. A fungus having resistance to gastric acid or bile acid is one that can survive under general conditions of the digestive organ and reach the intestine. In general, the pH of the interior of the stomach of a human or an animal may reach 2 or less when the stomach is empty, but if a food is administered, the pH of the interior of the stomach is within a range of 3.5 to 6, which is higher than that when the stomach is empty. Therefore, strains having acid resistance, which can be used in the additive for animal feed of the present invention, can be obtained by, for example, selecting bacterial strains that can survive in a source for isolation treated at about pH 3.5 for 2 hours. Moreover, the selected fungus is treated in the presence of 10 g/l deoxycholic acid for about 24 hours, and a strain that is still alive is selected, to thereby yield a strain that has resistance to gastric acid or bile acid, and is suitable for the additive for animal feed of the present invention. Arrival of a living fungus to the intestine can be confirmed by, for example, determining the concentration of fungus bodies in feces of an animal.

The additive for animal feed of the present invention may comprise the above-mentioned fungi singly or in combination of two or more. The additive particularly preferably contains at least one of *Aspergillus sojae* and *Aspergillus oryzae.*

The concentration of fungus bodies of *Aspergillus sojae, Aspergillus tamarii, Aspergillus foetidus, Aspergillus niger,* and *Aspergillus oryzae* comprised in the additive for animal feed of the present invention is not particularly limited as long as the fungus is not killed in the digestive organ when the additive is administered to an animal. In general, the fungus bodies are cultured at appropriate concentrations for production of a feed additive having acidic enzyme activity and used to appropriately adjust the acidic enzyme activity in a feed additive.

The acidic enzyme contained in an additive for animal feed of the present invention is preferably a digestive enzyme produced by the above-mentioned fungi and is not particularly limited as long as the enzyme is not deactivated and has the activity under acidic conditions in the stomach and intestines. In particular, preferable is an enzyme having an optimal pH of 2. 5 to 5. 5. Examples thereof include acid α-amylase, glucoamylase, takadiastase, protease, cellulase, ribonuclease, nuclease, xylanase, pectinase, and lipase, and the additive for animal feed of the present invention contains one or two or more of them. In particular, the additive preferably contains acid amylase capable of degrading starch, which is one of main components of feeds for livestock animals. The acid amylase contained in the additive for animal feed of the present invention is not particularly limited as long as the acid amylase is an acidic enzyme capable of hydrolyzing starch, and examples thereof include α-amylase, β-amylase, and glucoamylase. Of those, acid-resistant α-amylase having an optimal pH of 3 is preferable.
The additive for animal feed of the present invention may contain at least one of acidic enzymes produced by *Aspergillus sojae, Aspergillus tamarii, Aspergillus foetidus, Aspergillus niger,* and *Aspergillus oryzae,* and the additive may further contain an enzyme derived from another fungus or an enzyme derived from another organism.

The acidic enzyme content in the additive for animal feed of the present invention may be appropriately adjusted depending on the species, body weights, or the like of animals. In general, in the case where the acidic enzyme activities are measured according to the solid Koji analysis method defined in the National Tax Agency Japan prescribed analysis method (National Tax Agency first instruction, third revision), the total of acidic enzyme activities per g of an additive for animal feed is preferably 12, 000 U or more, more preferably 20,000 U or more. In particular, the acid amylase activity per g of an additive for animal feed is preferably 100 U or more, more preferably 300 U or more. Meanwhile, the total of the acid protease activity and the acid carboxypeptidase activity is preferably 10,000 U or more, more preferably 15,000 U or more.

The additive for animal feed of the present invention can be obtained by culturing at least one of the fungi of above-mentioned *Aspergillus sojae, Aspergillus tamarii, Aspergillus foetidus, Aspergillus niger,* and *Aspergillus oryzae* to produce acidic enzymes. *Aspergillus sojae, Aspergillus tamarii, Aspergillus foetidus, Aspergillus niger,* and *Aspergillus oryzae* to be used in the additive for animal feed of the present invention can produce an acidic enzyme by culturing the fungi under general culture conditions, the fungi can produce acidic enzymes. For example, the fungi may be cultured at a temperature of 25°C to 40°C, but in general, the fungi are preferably cultured at 28 to 32°C. Meanwhile, the culture may be performed by a liquid culture method through reciprocating shaking culture or a jar fermenter culture or the like, or a solid culture method. The solid culture method is preferably used in the present invention because some of acidic enzyme-producing genes of the above-mentioned fungi may be expressed only in solid media.

Components of a medium to be used for culturing the fungi may be animal-derived or plant-derived components, but the medium preferably contains a plant-derived nutrient, preferably brown rice, bran, rice bran, soybean, or barley, for example. Of those, brown rice is particularly preferably contained as a nutrient. This may enhance the production efficiency of an acidic enzyme such as acid amylase.
The medium further contains: another carbon source such as a sugar including glucose, sucrose, or molasses; and another nitrogen source such as ammonia, an ammonium salt including ammonium sulfate, ammonium chloride, and ammonium nitrate, or a nitrate.

A culture thus obtained may be used without modification as the additive for animal feed of the present invention, or a part containing fungus bodies and an acidic enzyme may be separated from the culture and added to an additive for animal feed. For example, in the case where fungus bodies are cultured using a solid medium, the fungus bodies are preferably pulverized together with the medium and added to an additive for animal feed from the viewpoint of convenience. Moreover, the culture is preferably processed by drying or by addition of an optional component for enhancing storage stability to improve quality stability of the product.

The drying may be performed by air drying, natural drying, spray drying, freeze drying, or the like, which is not particularly limited, and of those, air drying is preferable. Meanwhile, in the case of employing freeze drying, a protective agent may be added. The type of the protective agent is not particularly limited, but it is preferable to use one or two or more selected from skim milk, sodium glutamate, and a sugar. In the case of using a sugar, the type of the sugar is not particularly limited, but glucose or trehalose is preferably used.
After the drying, preferably, the resultant dried product is placed in a gas-barrier aluminum bag together with a deoxidizer and a dehydrator, and the bag is sealed and stored at a temperature from room temperature to low temperatures. This can store fungus bodies alive for a long period of time.

The additive for animal feed of the present invention preferably contains Kojic acid at a low concentration. In general, the concentration of Kojic acid is preferably 0.1 mg/l or less, more preferably 0.01 mg/l or less.

Meanwhile, the additive for animal feed of the present invention may be mixed with a component of a general animal feed to prepare a feed for promoting animal growth. In the case where the above-mentioned *Aspergillus* fungi further have antibacterial activity against a pathogen that causes an intestinal infection of an animal and/or protozoa-killing activity against a coccidium, a feed for preventing/treating the intestinal infection of the animal caused by the pathogen and/or coccidium can be obtained.
The type and components of the feed are not particularly limited as long as fungus bodies contained in an additive for animal feed of the present invention are not killed, and acidic enzymes are not deactivated. In general, the additive can be added to animal feeds such as feeds for livestock animal, pet foods, and supplements for animal.

The feed of the present invention can be manufactured by adding the additive for animal feed of the present invention to a component of a feed. The concentration of the additive for animal feed contained in the feed of the present invention is not particularly limited and may be appropriately adjusted depending on the animal's species, body weight, age, sex, intended use, health condition, feed component, etc., and is generally 10 to 5,000 ppm, preferably 50 to 1,000 ppm of the total amount of the feed based on a dry form.

The additive for animal feed of the present invention may be added to and mixed in a feed component without modification, but in the case of addition and mixing of a powdery or solid additive for animal feed, the additive may be modified before use into a liquid or a gel for the purpose of facilitating the mixing in a feed. In this case, water; a vegetable oil such as soybean oil, rapeseed oil, or corn oil; a liquid animal oil; and a water-soluble polymer compound such as polyvinylalcohol, polyvinylpyrrolidone, or polyacrylic acid may be used as a liquid carrier. Meanwhile, in order to keep the uniformity of the concentration of fungus bodies in a feed, the feed preferably contains a water-soluble polysaccharide such as alginic acid, sodium alginate, a xanthan gum, casein sodium, an arabic rubber, a guar gum and tamarind seed polysaccharide. Moreover, for the purpose of preventing proliferation of saprophyts, an organic acid may be blended to acidify a liquid viable cell agent.

Examples of the species of animals that ingest the feed of the present invention include, but not limited to, mammals, birds, reptiles, amphibians, and fishes. The feed can be particularly preferably used for fowls and domestic animals. The amount of a feed ingested by an animal may be appropriately adjusted depending on the animal's species, body weight, age, sex, intended use, health condition, feed component, etc.

### Examples

### (1-1) Selection of acid-resistant and bile acid-resistant Aspergillus fungi

A potato dextrose agar medium was adjusted to pH 5 and sterilized at 121°C for 15 minutes. Sodium deoxycholate was added to the medium at a concentration of 10 g/L at the time when the temperature of the agar medium was lowered to 60°C, and *Aspergillus* fungi stored at Akita Konno Co. , Ltd. (248 Kariwano, Daisen-shi, Akita-ken, Japan) were inoculated thereto. As a result, *Aspergillus sojae, Aspergillus tamarii, Aspergillus foetidus,* and *Aspergillus niger* grew well on the medium. Of those, *Aspergillus sojae* AOK 210 strain, *Aspergillus tamarii* AOK 43 strain, *Aspergillus foetidus* AOK N4586 strain, and *Aspergillus niger* AOK B650 *strain* grew particularly well.

### (1-2) Selection of acid-resistant and bile acid-resistant Aspergillus fungi

In the same way as above, many *Aspergillus* fungi derived from various fermented foods were inoculated to a medium containing sodium deoxycholate, and a strain that showed the best growth performance was selected from strains that grew on the medium. The strain named *Aspergillus oryzae* IK-05074 strain and deposited at the International patent Organism Depositary, National Institute of Advanced Industrial Science and Technology.
The mycological properties of IK-05074 strain are as noted above.

### (2-1) Experiment for comparing strains

### (a) Culture of fungus bodies and Measurement of acidic enzyme activity

Brown rice was used as a solid medium to culture AOK 210 strain, AOK 43 strain, AOK N4586 strain, and AOK B650 strain, which were selected in the section (1-1). That is, 100 g of brown rice was allowed to swell by immersion in water for a full day and placed in a polycarbonate container (14 cm in diameter, 10 cm in depth) having a lid with a sterilized filter so that the height of the rice was 2 cm, followed by sterilization in an autoclave at 121°C for 15 minutes. Fungus bodies of the above-mentioned fugni were separately inoculated to the medium and cultured at 28°C for 5 days, to thereby produce inoculums.
Subsequently, brown rice allowed to swell in the same way as above was layered in a stainless steel vat with a size of 30 × 40 × 10 cm so that the height of the rice was 5 cm, and a lid covered with a 20 × 25 cm filter and having a vent hole was placed thereon, followed by sterilization in a large-scale autoclave at 121°C for 25 minutes. The vat was cooled, and the totals of the above-mentioned inoculums, which had been previously cultured, were inoculated thereto. The vat was placed in an incubator at 28°C to culture the inoculums for 7 days.
After the culture, the rice was air-dried at 35 °C and pulverized using a jet mill, to thereby yield solid cultures of the respective inoculums. Meanwhile, the same procedures were repeated for *Aspergillus kawachii* AOK 1006s strain (Akita Konno Co. , Ltd.), to thereby yield a solid culture.

The acid-resistant α-amylase activity and the total sum of acid protease activity and acid carboxypeptidase activity per g of each solid culture were measured. The results are shown in Table 1. The measurement of the above-mentioned acidic enzymes was performed according to the acid-resistant α-amylase activity measurement method, acid protease activity measurement method, and acid carboxypeptidase activity measurement method of the solid Koji analysis method defined in the National Tax Agency Japan prescribed analysis method (National Tax Agency first instruction, third revision).

### (b) Administration test

An administration test of the strains obtained above to chicks was performed. The solid cultures of AOK 210 strain, AOK 43 strain, AOK N4586 strain, or AOK B650 strain obtained above was respectively mixed in a feed (for SD broiler in early and late stage, manufactured by Nippon Formula Feed Mfg Co., Ltd.) at concentrations of 100 ppm and 150 ppm based on the total mass of the feed, and the resultant products were used in Examples 1 to 8. Meanwhile, the solid culture of AOK 1006s strain was mixed in the feed in the same way as above, and the resultant products were used in Comparative Examples 1 and 2. Moreover, brown rice, which had been sterilized in an autoclave and dried without adding a fungus, was mixed in the feed at a concentration of 150 ppm, and the resultant product was defined as control. In the administration test, chicks of the respective groups, each group consisting of 10 one-week-old chicks, were allowed to freely ingest the above-mentioned feeds for 28 days for fattening. The average body weights of 35-day-old chicks of the respective groups are shown in Table 1.

**[Table 1]**

| | Strain | Acid-resis tant α-amylase activity (U) | Total sum of acid protease activity and acid carboxypeptidase activity (U) | Addition amount (ppm) | Average body weight on day 35 (g) |
|---|---|---|---|---|---|
| Example 1 | *Aspergillus sojae* AOK 210 | 2,555 | 46,917 | 100 | 1,582 |
| Example 2 | *Aspergillus sojae* AOK 210 | | | 150 | 1,587 |
| Example 3 | *Aspergillus* 43 *tamarii* AOK | 313 | 50,248 | 100 | 1,507 |
| Example 4 | *Aspergillus tamarii* AOK 43 | | | 150 | 1,512 |
| Example 5 | *Aspergillus foetidus* AOK N4586 | 586 | 29,660 | 100 | 1,575 |
| Example 6 | *Aspergillus foetidus* AOK N4586 | | | 150 | 1,582 |
| Example 7 | *Aspergillus niger* AOK B650 | 399 | 20,341 | 100 | 1,562 |
| Example 8 | *Aspergillus niger* AOK B650 | | | 150 | 1,580 |
| Comparative Example 1 | *Aspergillus kawachii* AOK 1006s | 74 | 8,194 | 100 | 1,422 |
| Comparative Example 2 | *Aspergillus kawachii* AOK 1006s | | | 150 | 1,428 |
| Control | - | - | - | 150 | 1,405 |

In the cases of the solid cultures of AOK 210 strain, AOK 43 strain, AOK N4586 strain, and AOK B650 strain, each acid-resistant α-amylase activity was found to be at least 4-fold higher than that of the culture obtained by culturing AOK 1006s strain. Meanwhile, the total sums of acid protease activity and acid carboxypeptidase activity were found to be about 2.5 to 6-fold larger than that of the culture of AOK 1006s. The results revealed that the above-mentioned four fungi had excellent abilities to produce acid α-amylase, acid protease, and acid carboxypeptidase. In particular, AOK 210 strain was found to have excellent ability to produce all of acid α-amylase, acid protease, and acid carboxypeptidase.

The chicks of the groups of Examples 1 to 8, which had been administered with the feeds containing the additives for animal feed of the present invention, were found to gain much body weights than chicks of Comparative Examples 1 and 2. The results indicated that the feeds containing the additives for animal feed of the present invention have excellent effects of promoting the growth of animals. In particular, the feed containing the additive for animal feed produced using AOK 210 strain was found to have excellent effect of promoting weight gain of animals.

### (2-2) Experiment for comparing strains

### (a) Culture of fungus bodies and Measurement of acidic enzyme activity

Brown rice was used as a solid medium to culture IK-05074 strain selected in the section (1-2). The culture was performed in the same way as above except that an inoculum was produced by 10-day culture, and the total of the inoculum was inoculated and cultured for 10 days. Meanwhile, *Aspergillus kawachii* AOK 1006s strain was cultured in the same way as above. After the culture, the same procedures as above were repeated, to thereby yield a solid culture of the strain.

Subsequently, a potato dextrose liquid medium was sterilized at 121°C for 15 minutes, and sodium deoxycholate was added to the medium at a concentration of 5 g/L at the time when the temperature of the medium was lowered to 60°C. Then, IK-05074 strain was inoculated thereto and cultured at 28 °C for 6 days. The culture was filtrated using a 0.4 µm-filter, and the fungus bodies were collected. The collected fungus bodies were washed with the medium before culture three times to remove extracellular enzymes. Thereafter, medium components were added to the fungus bodies, and air-drying was performed at 36°C for 24 hours, to thereby yield a solid culture of IK-05074 strain containing no acidic enzyme.

The acid-resistant α-amylase activity and the total sum of acid protease activity and acid carboxypeptidase activity per g of each solid product were measured in the same way as the section (2-1) (a). The results are shown in Table 2.

### (b) Administration test

An administration test of IK-05074 strain to chicks was performed in the same way as the section (2-1) (b). The solid culture of IK-05074 strain obtained above was mixed in the feed at concentrations of 100 ppm and 150 ppm, and the resultant products were used in Examples 9 and 10. Meanwhile, the solid culture of *Aspergillus kawachii* was mixed in the feed in the same way as above, and the resultant products were used in Comparative Examples 3 and 4; and the solid culture of IK-05074 strain with acidic enzyme removed was mixed in the feed at a concentration of 150 ppm, and the resultant product was used in Comparative Example 5. Moreover, brown rice, which had been sterilized in an autoclave and dried without adding a fungus, was mixed in the feed at a concentration of 150 ppm, and the resultant product was defined as control. In the administration test, chicks of the respective groups, each group consisting of 10 one-week-old chicks, were allowed to freely ingest the above-mentioned feeds for 48 days for fattening. The average body weights of 55-day-old chicks of the respective groups are shown in Table 2.

**[Table 2]**

| | Strain | Acid-resistant α-amylase activity | Total sum of acid protease activity and acid carboxypeptidase activity (U) | Additi on amount (ppm) | Average body weight on day 55 (g) |
|---|---|---|---|---|---|
| Example 9 | A.oryzae IK-05074 | | | 100 | 5,430 |
| Example 10 | A.oryzae IK-05074 | 4,208 | 51,280 | 150 | 6,120 |
| Comparative Example 3 | A.Kawachii AOK 1006s | | | 100 | 5,120 |
| Comparative Example 4 | A.Kawachii AOK 1006s | 74 | 8,316 | 150 | 5,340 |
| Comparative Example 5 | A.oryzae IK-05074 (acidic enzyme removed) | <10 | <2,000 | 150 | 5,390 |
| Control | - | - | - | 150 | 4,960 |

The acid-resistant α-amylase activity of the culture obtained by culturing *Aspergillus oryzae* IK-05074 strain was found to be about 57-fold larger than that of the culture obtained by culturing *Aspergillus kawachii.* Meanwhile, the total sum of acid protease activity and acid carboxypeptidase activity was found to be about 6-fold larger than that of the culture of *Aspergillus kawachii.* The results revealed that IK-05074 strain had an excellent ability to produce acid amylase, acid protease, and acid carboxypeptidase, in particular, acid amylase.

The chicks of Examples 9 and 10, which had been administered with the feeds containing the additive for animal feed of the present invention, were found to gain much body weights than Comparative Examples 3 to 5. The results suggest that the feeds containing the additive for animal feed of the present invention, which contains *Aspergillus oryzae* and an acidic enzyme produced by the fungus, have an effect of promoting the growth of animals.

### (3) Experiment for comparing solid mediums

### (a) Culture of fungus bodies and measurement of acid-resistant α-amylase activity

Brown rice, barley, and pulverized soybean were used as solid mediums to culture AOK 210 strain. That is, 100 g of each of brown rice, barley, and pulverized soybean was allowed to swell by immersion in water overnight and separately layered in stainless steel vats with a size of 30 × 40 × 5 cm so that the height of each medium was 2.0 cm, and the surface of the vat was covered with a filter paper. Then, a stainless steel lid was placed thereon, followed by sterilization in a large-scale autoclave at 121°C for 30 minutes. The vats were cooled, and the totals of the inoculums cultured in the section (2-1) (a) were separately inoculated to the media. The vats were placed in a 28°C-incubator to culture the inoculums for 5 days.
After the culture, the resultant cultures were air-dried at 35°C and pulverized using a jet mill, to thereby yield solid cultures. For each of the solid cultures, the acid-resistant α-amylase activity was measured. The results are shown in Table 3. The measurement of the acid-resistant α-amylase activity was performed in the same way as above.

### (b) Administration test

Each of administration test of the dried solid cultures obtained above to chicks was performed. Each of the solid cultures was mixed in a feed (for SD broiler in early and late stage, manufactured by Nippon Formula Feed Mfg Co. , Ltd.) at concentrations of 50 ppm and 100 ppm of the total mass of the feed, and the resultant products were used in Examples 11 to 16. Moreover, brown rice, which had been sterilized in an autoclave and dried without adding a fungus, was mixed in the feed at a concentration of 100 ppm, and the resultant product was defined as control. In the administration test, chicks of the respective groups, each group consisting of 10 one-week-old chicks, were allowed to freely ingest the above-mentioned feeds for 35 days for fattening. The average body weights of 42-day-old chicks of the respective groups are shown in Table 3.

**[Table 3]**

| | Type of medium | Acid-resistant α-amylase activity (U) | Addition amount (ppm) | Average body weight on day 42 (g) |
|---|---|---|---|---|
| Example 11 | Brown rice | 2,606 | 50 | 1,690 |
| Example 12 | Brown rice | | 100 | 1,708 |
| Example 13 | Barley | 1,517 | 50 | 1,595 |
| Example 14 | Barley | | 100 | 1,663 |
| Example 15 | Soybean | 1,723 | 50 | 1,588 |
| Example 16 | Soybean | | 100 | 1,650 |
| Control | Brown rice | - | 100 | 1,465 |

The acid-resistant α-amylase activity of the culture obtained by using brown rice as a solid medium component was found to be obviously higher (about 1.5-fold) than that of the culture obtained using soybean or barley as a solid medium component. The results suggest that, if brown rice is used as a solid medium component, the ability to produce acid amylase is particularly enhanced.

The body weights of the chicks of the groups of Examples 11 to 16, which had been administered with the feeds comprising the additives for animal feed of the present invention, were found to gain much body weights than chicks of the control. In particular, the feed of the present invention, which comprises an additive for animal feed produced by using brown rice as a solid medium component, was found to have an excellent effect of promoting weight gain.

### (4-1) Measurement of antibacterial activity of culture of Aspergillus fungus

AOK 210 strain, AOK 43 strain, AOK N4586 strain, and AOK B650 strain were cocultured with a pathogen to test the antibacterial activities of the solution against the pathogen.

*Salmonella enteritidis* (SE) was aerobically cultured on a standard agar medium at 37°C for 24 hours. Colonies that grew on the plate were scraped off and suspended in sterilized physiological saline. 500 ml of brain heart infusion broth "Nissui" was prepared in a 1 L-volume conical flask and sterilized in an autoclave, and SE was aseptically added thereto so that the final concentration of SE was about 1.0 × 10⁴ to 1.0 × 10⁵ CFU/ml. To the conical flask was aseptically added 5 g of each of the pulverized solid cultures of AOK 210 strain, AOK 43 strain, AOK N4586 strain, and AOK B650 strain obtained in the section (2-1) (a), and the resultant products were used in Examples 17 to 20. A conical flask to which 5 g of the pulverized solid culture of AOK 1006s strain was aseptically added was used in Comparative Example 6, and a conical flask containing no Koji mold culture was defined as control. The solution in the conical flasks were cultured with gentle stirring under aerobic conditions in a 37°C-incubator.

*Clostridium perfringens* (CP) was anaerobically cultured on egg yolk-supplemented CW agar medium (manufactured by Nissui Pharmaceutical Co., Ltd.) using Anaero Pack Kenki (manufactured by Mitsubishi Gas Chemical Company, Inc.) at 37°C for 24 hours. Colonies that grew on the plate were scraped off and suspended in sterilized physiological saline. 500 ml of brain heart infusion broth "Nissui" was prepared in a 1 L-volume conical flask and sterilized in an autoclave, and CP was aseptically added thereto so that the final concentration of CP was about 1.0 × 10⁴ to 1.0 × 10⁵ CFU/ml. To the conical flask was aseptically added 5 g of each of the pulverized solid cultures of AOK 210 strain, AOK 43 strain, AOK N4586 strain, and AOK B650 strain obtained in the section (2-1) (a), and the resultant products were defined as feeds of Examples 21 to 24. A conical flask to which 5 g of the pulverized solid culture of AOK 1006s strain was aseptically added was used in Comparative Example 7, and a conical flask containing no Koji mold culture was defined as control. The solution in the conical flasks were cultured with gentle stirring using Anaero Pack Kenki under anaerobic conditions in a 37°C-incubator.

The SE and CP viable cells were counted 0, 3, and 7 days after the start of the test. A method of counting SE viable cells includes the steps of: serially diluting a collected culture medium 10-fold with sterilized physiological saline; applying 0.1 ml of each of the diluted solutions on X-SAL agar medium "Nissui"; aerobically culturing the bacterium at 37°C for 24 hours; and counting characteristic colonies that grew on the medium. Amethodof counting CP viable cells includes the steps of: serially diluting a collected culture medium 10-fold with sterilized physiological saline; applying 0.1 ml of' each of the diluted solutions on egg yolk-supplemented CW agar medium (manufactured by Nissui Pharmaceutical Co., Ltd.); anaerobically culturing the bacterium using Anaero Pack Kenki at 37°C for 24 hours; and counting characteristic colonies that grew on the medium.

Meanwhile, in order to simultaneously determine the concentration of Kojic acid, the culture medium was centrifuged at 8, 000 rpm for 10 minutes, and the supernatant was filtered through a cellulose-mixed ester type membrane filter (pore size 0.45 µm, manufactured by ADVANTEC MFS, INC.). Waters 600 (Multisolvent Delivery system) and waters 490E (Programmable multiwavelength Detector) were used as HPLC apparatus. A column used was YMC-Pack ODS-AM 6.0 mm × 150 mm (manufactured by YMC Co. , Ltd.). Detection of Kojic acid was performed at a measurement wavelength of 270 nm using a mobile phase of 0.1mol/l sodiumdihydrogen phosphate solution (pH 3.0)-methanol (97 : 3) at a flow rate of 1.0 ml/min and a column temperature of 40°C. The calibration curve was created by using Kojic acid (guaranteed reagent, manufactured by Wako Pure Chemical Industries, Ltd.).
Table 4 shows the numbers of SE viable cells, Table 5 shows the concentrations of Kojic acid in cultures obtained by the SE-coculture test, Table 6 shows the numbers of CP viable cells, and Table 7 shows the concentrations of Koj ic acid in cultures obtained by the CP-coculture test.

**[Table 4]**

| | | Number of SE viable cells (CFU/ml) | | |
|---|---|---|---|---|
| | | Day 0 of test | Day 3 of test | Day 7 of test |
| Example 17 | SE + *Aspergillus sojae* AOK 210 | 5.0×10⁴ | <1.0×10² | <1.0×10² |
| Example 18 | SE + *Aspergillus tamarii* AOK 43 | 5.0×10⁴ | 2.2×10³ | <1.0×10² |
| Example 19 | SE + *Aspergillus foetidus* AOK N4586 | 5.0×10⁴ | 3.9×10³ | 6.9×10² |
| Example 20 | SE + *Aspergillus niger* AOK B650 | 5.0×10⁴ | 6.1×10³ | 8.4×10² |
| Comparative Example 6 | SE + *Aspergillus kawachii* AOK 1006s | 5.0×10⁴ | 2.4×10⁸ | 2.8×10⁶ |
| Control | Only SE | 5.0×10⁴ | 2.5×10⁸ | 2.8×10⁸ |

**[Table 5]**

| | | Kojic acid content (mg/L) | | |
|---|---|---|---|---|
| | | Day 0 of test | Day 3 of test | Day 7 of test |
| Example 17 | SE + *Aspergillus sojae* AOK 210 | <0.1 | <0.1 | <0.1 |
| Example 18 | SE + *Aspergillus tamarii* AOK 43 | <0.1 | <0.1 | <0.1 |
| Example 19 | SE + *Aspergillus foetidus* AOK N4586 | <0.1 | <0.1 | <0.1 |
| Example 20 | SE + *Aspergillus niger* AOK B650 | <0.1 | <0.1 | <0.1 |
| Comparative Example 6 | SE + *Aspergillus kawachii* AOK 1006s | <0.1 | <0.1 | <0.1 |
| Control | Only SE | <0.1 | <0.1 | <0.1 |

**[Table 6]**

| | | Number of CP viable cells (CFU/ml) | | |
|---|---|---|---|---|
| | | Day 0 of test | Day 3 of test | Day 7 of test |
| Example 21 | CP + *Aspergillus sojae* AOK 210 | 9.6×10⁴ | <1.0×10² | <1.0×10² |
| Example 22 | CP + *Aspergillus tamarii* AOK 43 | 9.6×10⁴ | 3.9×10⁴ | 7.2×10³ |
| Example 23 | CP+ *Aspergillus foetidus* AOK N4586 | 9.6×10⁴ | 6.2×10⁴ | 5.9×10³ |
| Example 24 | CP + *Aspergillus niger* AOK B650 | 9.6×10⁴ | 4.4×10⁴ | 5.0×10³ |
| Comparative Example 7 | CP + *Aspergillus kawachii* AOK 1006s | 9.6×10⁴ | 3.2×10⁵ | 5.2×10⁴ |
| Control | Only CP | 9.6×10⁴ | 4.8×10⁵ | 1.0×10⁶ |

**[Table 7]**

| | | Kojic acid content (mg/L) | | |
|---|---|---|---|---|
| | | Day 0 of test | Day 3 of test | Day 7 of test |
| Example 21 | CP + *Aspergillus sojae* AOK 210 | <0.1 | <0.1 | <0.1 |
| Example 22 | CP + *Aspergillus tamarii* AOK 43 | <0.1 | <0.1 | <0.1 |
| Example 23 | CP + *Aspergillus foetidus AOK N4586* | <0.1 | <0.1 | <0.1 |
| Example 24 | CP + *Aspergillus niger* AOK B650 | <0.1 | <0.1 | <0.1 |
| Comparative Example 7 | Cp + *Aspergillus kawachii* AOK 1006s | <0.1 | <0.1 | <0.1 |
| Control | Only CP | <0.1 | <0.1 | <0.1 |

As shown in Examples 17 to 20, the solid cultures of AOK 210 strain, AOK 43 strain, AOK N4586 strain, and AOK B650 strain were found to have antibacterial activities against SE, which are obviously higher compared with the solid culture of AOK 1006s strain shown in Comparative Example 6. In particular, in the case of coculture with AOK 210 strain, the number of SE viable cells fell below the detection limit on day 3 of the test, and the antibacterial activity was found to be the highest. Meanwhile, in the case of AOK 43 strain, the number of SE viable cells fell below the detection limit on day 7 of the test. In the case of control, the number of viable cells was raised by day 7 of the test and reached 2.8 × 10⁸ CFU/ml on day 7.
In addition, the Kojic acid contents in all the suspensions were found to be below the detection limit.

As shown in Examples 21 to 24, the solid cultures of AOK 210 strain, AOK 43 strain, AOK N4586 strain, and AOK B650 strain were found to have antibacterial activities against CP, which are obviously higher compared with the solid culture of AOK 1006s strain shown in Comparative Example 7. In particular, in the case of coculture with AOK 210 strain, the number of CP viable cells fell below the detection limit on day 3 of the test, and the antibacterial activity was found to be the highest. In the case of control, the number of viable cells was raised by day 7 of the test and reached 1.0 × 10⁶ CFU/ml on day 7.
In addition, the Kojic acid contents in all the suspensions were found to be below the detection limit.

*Escherichia coli* (EC) was aerobically cultured on a standard agar medium (manufactured by Nissui Pharmaceutical Co., Ltd.) at 37°C for 24 hours. Colonies that grew on the plate were scraped off and suspended in sterilized physiological saline. 500 ml of brain heart infusion broth "Nissui" (manufactured by Nissui Pharmaceutical Co. , Ltd.) was prepared in a 1 L-volume conical flask and sterilized in an autoclave, and EC was aseptically added thereto so that the final concentration of EC was about 1.0 × 10⁵ to 1.0 × 10⁶ CFU/ml. To the conical flask was aseptically added 5 g of each of the pulverized solid cultures of AOK 210 strain, AOK 43 strain, AOK N4586 strain, and AOK B650 strain obtained in the section (2-1) (a), and the resultant products were used in Examples 25 to 28. A conical flask to which 5 g of the pulverized solid culture of AOK 1006s was aseptically added was used in Comparative Example 8, and a conical flask containing no Koji mold culture was defined as control. The solution in the conical flasks were cultured with gentle stirring under aerobic conditions in a 37°C-incubator.

*Staphylococcus aureus* (SA) was aerobically cultured on a standard agar medium at 37°C for 24 hours. Colonies that grew on the plate were scraped off and suspended in sterilized physiological saline. 500 ml of brain heart infusion broth "Nissui" (manufactured by Nissui Pharmaceutical Co., Ltd.) was prepared in a 1 L-volume conical flask and sterilized in an autoclave, and SA was aseptically added thereto so that the final concentration of SA was about 1.0 × 10⁵ to 1.0 × 10⁶ CFU/ml. To the conical flask was aseptically added 5 g of each of the pulverized solid cultures of AOK 210 strain, AOK 43 strain, AOK N4586 strain, and AOK B650 strain obtained in the section (2-1) (a), and the resultant products were defined as Examples 29 to 32. A conical flask to which 5 g of the pulverized solid culture of AOK 1006s was aseptically added was defined as Comparative Example 9, and a conical flask containing no Koji mold culture was defined as control. The solution in the conical flasks were cultured were cultured with gentle stirring under aerobic conditions in a 37°C-incubator.

The EC and SA viable cells were counted 0, 3, and 7 days after the start of the test. A method of counting EC viable cells includes the steps of: serially diluting a collected culture medium 10-fold with sterilized physiological saline; applying 0.1 ml of each of the diluted solutions on Chromocult coliform agar "Merck" (manufactured by Merck & Co., Inc.); aerobically culturing the bacterium at 37°C for 24 hours; and counting characteristic colonies that grew on the medium. A method of counting SA viable cells includes the steps of: serially diluting a collected culture medium 10-fold with sterilized physiological saline; applying 0.1 ml of each of the diluted solutions on egg yolk-supplemented mannitol salt agar "Eiken" (manufactured by Eiken Kizai Co., Ltd.); aerobically culturing the bacterium at 37°C for 48 hours; and counting characteristic colonies that grew on the medium.
Table 8 shows the numbers of EC viable cells, and Table 9 shows the numbers of SA viable cells.

**[Table 8]**

| | | Number of EC viable cells (CFU/ml) | | |
|---|---|---|---|---|
| | | Day 0 of test | Day 3 of test | Day 7 of test |
| Example 25 | EC + *Aspergillus sojae* AOK 210 | 1.4×10⁵ | <1.0×10² | <1.0×10² |
| Example 26 | EC + *Aspergillus tamarii* AOK 43 | 1.4×10⁵ | 1.7×10³ | <1.0×10² |
| Example 27 | EC + *Aspergillus foetidus* AOK N4586 | 1.4×10⁵ | 5.1×10³ | <1.0×10² |
| Example 28 | EC + *Aspergillus niger* AOK B650 | 1.3×10⁵ | 3.3×10³ | <1.0×10² |
| Comparative Example 8 | EC + *Aspergillus kawachii* AOK 1006s | 1.4×10⁵ | 4.1×10⁸ | 3.5×10⁵ |
| Control | Only EC | 1.4×10⁵ | 4.8×10⁸ | 1.0×10⁷ |

**[Table 9]**

| | | Number of SA viable cells (CFU/ml) | | |
|---|---|---|---|---|
| | | Day 0 of test | Day 3 of test | Day 7 of test |
| Example 29 | SA + *Aspergillus sojae* AOK 210 | 2.7×10⁵ | <1.0×10² | <1.0×10² |
| Example 30 | SA + *Aspergillus tamarii* AOK 43 | 2.5×10⁵ | 6.1×10³ | <1.0×10² |
| Example 31 | SA + *Aspergillus foetidus* AOK N4586 | 3.2×10⁵ | 5.8×10³ | <1.0×10² |
| Example 32 | SA + *Aspergillus niger* AOK B650 | 2.4×10⁵ | 7.3×10³ | <1.0×10² |
| Comparative Example 9 | SA + *Aspergillus kawachii* AOK 1006s | 2.7×10⁵ | 2.8×10⁷ | 5.2×10⁵ |
| Control | Only SA | 2.5×10⁵ | 4.2×10⁸ | 7.2×10⁷ |

As shown in Examples 25 to 28, the solid cultures of AOK 210 strain, AOK 43 strain, AOK N4586 strain, and AOK B650 strain were found to have antibacterial activities against EC, which are obviously higher compared with the solid culture of AOK 1006s strain shown in Comparative Example 8. In particular, in the case of coculture with AOK 210 strain, the number of EC viable cells fell below the detection limit on day 3 of the test, and the antibacterial activity was found to be the highest. Meanwhile, in the cases of AOK 43 strain, AOK N4586 strain, and AOK B650 strain, each of the number of EC viable cells fell below the detection limit on day 7 of the test. In the case of control, the number of viable cells was raised by day 7 of the test and reached 1.0 × 10⁷ CFU/ml on day 7.

As shown in Examples 29 to 32, the solid cultures of AOK 210 strain, AOK 43 strain, AOK N4586 strain, and AOK B650 strain were found to have antibacterial activities against SA, which are obviously higher compared with AOK 1006s strain shown in Comparative Example 9. In particular, in the case of coculture with AOK 210 strain, the number of SA viable cells fell below the detection limit on day 3 of the test, and the antibacterial activity was found to be the highest. Meanwhile, in the cases of AOK 43 strain, AOK N4586 strain, and AOK B650 strain, each of the number of SA viable cells fell below the detection limit on day 7 of the test. In the case of control, the number of viable cells was raised by day 7 of the test and reached 7.2 × 10⁷ CFU/ml on day 7.

### (4-2) Measurement of antibacterial activity of culture of Aspergillus oryzae

IK-05074 strain was cocultured with a pathogen to test the antibacterial activity of IK-05074 strain against the pathogen in the same way as the section (4-1).

A conical flask containing *Salmonella enteritidis* (SE) and the pulverized solid culture of IK-05074 strain obtained in the section (2-2) (a) was used in Example 33, a conical flask containing SE and the pulverized solid culture of AOK 1006s strain was used in Comparative Example 10, and a conical flask containing no Koji mold culture was defined as control.

A conical flask containing *Clostridium perfrigens* (CP) and the pulverized solid culture of IK-05074 strain obtained in the section (2-2) (a) was defined as Example 34, a conical flask containing CP and the pulverized solid culture of AOK 1006s strain was defined as Comparative Example 11, and a conical flask containing no Koji mold culture was defined as control.

The SE and CP viable cells were counted 0, 3, and 7 days after the start of the test. In addition, each of the concentration of Kojic acid was quantified at the same time.
Table 10 shows the numbers of SE viable cells, Table 11 shows the concentrations of Kojic acid in cultures obtained by the SE-coculture test, Table 12 shows the numbers of CP viable cells, and Table 13 shows the concentrations of Kojic acid in cultures obtained by the CP-coculture test.

**[Table 10]**

| | | Number of SE viable cells (CFU/ml) | | |
|---|---|---|---|---|
| | | Day 0 of test | Day 3 of test | Day 7 of test |
| Example 33 | SE + *Aspergillus oryzae* IK-05074 | 4.5×10⁴ | <1.0×10² | <1.0×10² |
| Comparative Example 10 | SE + *Aspergillus kawachii* AOK 1006s | 4.5×10⁴ 4.5×10⁴ | 3.1×10⁸ 3.1×10⁸ | 2.6×10⁶ 2.6×10⁶ |
| Control | Only SE | 4.5×10⁴ | 3.3×10⁸ | 2.7×10⁸ |

**[Table 11]**

| | | Kojic acid content (mg/L) | | |
|---|---|---|---|---|
| | | Day 0 of test | Day 3 of test | Day 7 of test |
| Example 33 | SE + *Aspergillus oryzae* IK-05074 | <0.1 | <0.1 | <0.1 |
| Comparative Example 10 | SE + *Aspergillus kawachii* AOK 1006s | <0.1 | <0.1 | <0.1 |
| Control | Only SE | <0.1 | <0.1 | <0.1 |

**[Table 12]**

| | | Number of CP viable cells (CFU/ml) | | |
|---|---|---|---|---|
| | | Day 0 of test | Day 3 of test | Day 7 of test |
| Example 34 | CP + *Aspergillus oryzae* IK-05074 | 5.0×10⁴ | <1.0×10² | <1.0×10² |
| Comparative Example 11 | CP + *Aspergillus kawachii* AOK 1006s | 5.0×10⁴ | 2.1×10⁵ | 3.4×10⁴ |
| Control | Only CP | 5.0×10⁴ | 6.1×10⁵ | 1.3×10⁶ |

**[Table 13]**

| | | Kojic acid content (mg/L) | | |
|---|---|---|---|---|
| | | Day 0 of test | Day 3 of test | Day 7 of test |
| Example 34 | CP + *Aspergillus oryzae* IK-05074 | <0.1 | <0.1 | <0.1 |
| Comparative Example 11 | CP + *Aspergillus kawachii* AOK 1006s | <0.1 | <0.1 | <0.1 |
| Control | Only CP | <0.1 | <0.1 | <0.1 |

As shown in Example 33, in the case of coculture of SE with IK-05074 strain, the number of SE viable cells fell below the detection limit on day 3 of the test, and the solid culture of IK-05074 strain was found to have antibacterial activity against SE, which is obviously higher compared with the solid culture of AOK 1006s strain shown in Comparative Example 10. In the case of control, the number of viable cells was raised after the start of the test and reached 3.3 × 10⁸ CFU/ml on day 3.
In addition, the Kojic acid contents in all the suspensions were found to be below the detection limit.

As shown in Example 34, in the case of coculture of CP with IK-05074 strain, the number of CP viable cells fell below the detection limit on day 3 of the test, and the solid culture of IK-05074 strain was found to have antibacterial activity against CP, which is obviously higher compared with the solid culture of AOK 1006s strain shown in Comparative Example 11. In the case of control, the number of fungus bodies was raised by day 7 and reached 1.3 × 10⁶ CFU/ml on day 7.
In addition, the Kojic acid contents in all the suspensions were found to be below the detection limit.

A conical flask containing *Escherichia coli* (EC) and the pulverized solid culture of IK-05074 strain obtained in the section (2-2) (a) was used in Example 35, a conical flask containing EC and the pulverized solid culture of AOK 1006s strain was defined as Comparative Example 12, and a conical flask containing no Koji mold culture was defined as control.

A conical flask containing *Staphyorococcus aureus* (SA) and the pulverized solid culture of IK-05074 strain obtained in the section (2-2) (a) was defined as Example 36, a conical flask containing CP and the pulverized solid culture of AOK 1006s strain was defined as Comparative Example 13, and a conical flask containing no Koji mold culture was defined as control.

The EC and SA viable cells were counted 0, 3, and 7 days after the start of the test. Table 14 shows the numbers of EC viable cells, and Table 15 shows the numbers of SA viable cells.

**[Table 14]**

| | | Number of EC viable cells (CFU/ml) | | |
|---|---|---|---|---|
| | | Day 0 of test | Day 3 of test | Day 7 of test |
| Example 35 | EC + *Aspergillus oryzae* IK-05074 | 1.0×10⁵ | <1.0×10² | <1.0×10² |
| Comparative Example 12 | EC + *Aspergillus kawachii* AOK 1006s | 1.0×10⁵ | 2.9×10⁸ | 5.2×10⁵ |
| Control | Only EC | 1.0×10⁵ | 4.0×10⁸ | 1.9×10⁷ |

**[Table 15]**

| | | Number of SA viable cells (CFU/ml) | | |
|---|---|---|---|---|
| | | Day 0 of test | Day 3 of test | Day 7 of test |
| Example 36 | SA + *Aspergillus oryzae* IK-05074 | 2.5×10⁵ | <1.0×10² | <1.0×10² |
| Comparative Example 13 | SA + *Aspergillus kawachii* AOK 1006s | 2.5×10⁵ | 3.9×10⁷ | 2.9×10⁵ |
| Control | Only SA | 2.5×10⁵ | 3.7×10⁸ | 6.9×10⁷ |

As shown in Example 35, in the case of coculture of EC with IK-05074 strain, the number of EC viable cells fell below the detection limit on day 3 of the test, and the solid culture of IK-05074 strain was found to have antibacterial activity against EC, which is obviously higher compared with the solid culture of AOK 1006s strain shown in Comparative Example 12. In the case of control, the number of viable cells was raised after the start of the test and reached 4.0 × 10⁸ CFU/ml on day 3.

As shown in Example 36, in the case of coculture of SA with IK-05074 strain, the number of SA viable cells fell below the detection limit on day 3 of the test, and the solid culture of IK-05074 strain was found to have antibacterial activity against SA, which is obviously higher compared with the solid culture of AOK 1006s strain shown in Comparative Example 13. In the case of control, the number of viable cells was raised after the test and reached 3.7 × 10⁸ CFU/ml on day 3.

### (5-1) Challenge study of Salmonella enteritidis to chick

Feeds obtained by mixing the pulverized solid cultures of AOK 210 strain, AOK 43 strain, AOK N4586 strain, and AOK B650 strain obtained in the section (2-1) (a) in each of feed of a chick (for SD broiler in early stage, manufactured by Nippon Formula Feed Mfg Co., Ltd., a feed supplemented with no antibacterial substance) at a concentration of 100 ppm of the total mass of the feed were used in Examples 37 to 40, respectively. Chicks of the respective groups, each group consisting of 12 chicks hatched from hatching eggs derived from broiler chickens (name: Chunky), were allowed to ingest the feeds of Examples 37 to 40 for 14 days. On the other hand, a feed obtained by mixing the pulverized solid culture of AOK 1006s strain in the feed at a concentration of 100 ppm was used in Comparative Example 14. A feed obtained by mixing lactose instead of a Koji mold culture in the feed at a concentration of 100 ppm was defined as control and used in the test in the same way as above. Each 7-day-old chick was orally challenged by *Salmonella enteritidis* (SE) at 1.8 × 10⁵ CFU. A SE strain isolated from the cecal content of a chicken that had died at a farm of a poultry producer in Gunma-ken was used for the challenge. From 14-day-old chicks, cecal contents were collected, and feces were collected by wiping the cloacae with swabs.

The SE viable cells in the cecal contents were counted by the following method to calculate infection indices and defense indices.
1 g of each cecal content was diluted 10-fold with sterilized phosphate buffered saline, and the solution was sufficiently mixed to prepare a sample stock solution. Subsequently, the sample stock solution was serially diluted 10-fold with sterilized physiological saline to prepare serially-diluted solutions. The sample stock solution and serially-diluted solutions were separately smeared on an SS agar plate medium "Nissui" (manufactured by Nissui Pharmaceutical Co., Ltd.) and a brilliant green agar plate medium (manufactured by Difco Laboratories), respectively, in an amount of 0.1 ml and cultured at 37°C for 24 hours, and the number of typical SE colonies grown on each plate medium was determined. Moreover, bacteria were collected from the colonies and inoculated to SIM agar medium "Nissui" (manufactured by Nissui Pharmaceutical Co., Ltd.) and TSI agar medium "Nissui" (manufactured by Nissui Pharmaceutical Co., Ltd.) each for a lysine decarboxylase test, and the media were cultured at 37°C for 24 hours to confirm their properties.
The number of SE viable cells per g of a cecal content was calculated by multiplying the number of colonies confirmed to be SE by a dilution rate of a diluted solution. Based on the results, infection indices and defense indices were calculated as follows. The infection index is a value indicating an infection rate of a pathogen, while the defense index is a value indicating an ability of each feed to protect the infection of a pathogen, which is calculated by compared with a feed containing no Koji mold culture.
Infection index: mean value of logarithms of the numbers of SE viable cells in cecal contents of the respective individuals (mean value of log CFU/g)
Defense index: infection index of control/infection index of each test group

For the feces collected from the cloacae, qualitative culture was performed for each individual by the following method to confirm the properties of SE. That is, the feces attached to swabs were suspended in 10 ml of sterilized phosphate buffered saline to prepare sample stock solutions, and the sample stock solutions was separately smeared on an SS agar plate medium and a brilliant green agar plate medium in an amount of 0.1 ml and cultured at 37 °C for 24 hours, and formation of typical SE colonies grown on each plate medium was estimated. Moreover, bacteria were collected from the colonies and inoculated to LIM agar medium "Nissui" (manufactured by Nissui Pharmaceutical Co., Ltd.), SIM agar medium, and TSI agar medium, and the media were cultured at 37 °C for 24 hours to confirm their properties.
The results are shown in Table 16.

**[Table 16]**

| | Number of viable cells in cecal content | | Number of individuals whose feces collected from cloacae contain SE Number of study chicks (12) |
|---|---|---|---|
| | Infection index | Defense index | |
| Example 37 | 3.4 | 2.3 | 5 |
| Example 38 | 4.8 | 1.6 | 10 |
| Example 39 | 5.1 | 1.5 | 9 |
| Example 40 | 4.6 | 1.7 | 11 |
| Comparative Example 14 | 7.8 | 1.0 | 12 |
| Control | 7.9 | - | 12 |

In the cases of the chicks administered with the feeds containing AOK 210 strain, AOK 43 strain, AOK N4586 strain, and AOK B650 strain of Examples 37 to 40, the concentrations of SE viable cells in the cecal contents were extremely low, and the SE infection indices were extremely low. In Example 37, the defense index was particularly high. In the case of chicks administered with the feed containing AOK 1006s of Comparative Example 14, the number of SE viable cells was almost the same as that of control. The results revealed that the cultures of the present invention containing acidic enzymes produced by *Aspergillus sojae, Aspergillus tamarii, Aspergillus foetidus,* and *Aspergillus niger* had effects of preventing infections caused by SE.

### (5-2) Challenge study of Salmonella enteritidis to chick

A feed obtained by mixing the pulverized solid culture of IK-05074 strain obtained in the section (2-2) (a) in a feed at a concentration of 100 ppm was used in Example 41, a feed obtained by mixing the pulverized solid culture of AOK 1006s strain in a feed at a concentration of 100 ppm was used in Comparative Example 15, and a feed obtained by mixing lactose instead of a Koji mold culture in a feed at a concentration of 100 ppm was defined as control. An SE challenge study was performed using the feeds to breed chicks. The study was performed in the same way as above except that each chick was orally challenged by SE in an amount of 2.0 × 10⁵ CFU.
The results are shown in Table 17.

**[Table 17]**

| | Number of viable cells in cecal content | | Number of individuals whose feces collected from cloacae contain SE Number of study chicks (12) |
|---|---|---|---|
| | Infection index | Defense index | |
| Example 41 | 3.1 | 2.5 | 5 |
| Comparative Example 15 | 7.5 | 1.0 | 12 |
| Control | 7.7 | - | 12 |

In the cases of the chicks administered with the feed containing IK-05074 strain of Example 41, the concentration of SE viable cells in the cecal content was extremely low, and the SE infection indexwas extremely low. In the case of chicks administered with the feed containing AOK 1006s of Comparative Example 15, the number of SE viable cells was almost the same as that of control.
The results revealed that the culture of the present invention containing an acidic enzyme produced by *Aspergillus oryzae* had an effect of preventing an infection caused by SE.

### (6-1) Challenge study of Clostridium perfringens to chick

Feeds obtained by mixing the pulverized solid cultures of AOK 210 strain, AOK 43 strain, AOK N4586 strain, and AOK B650 strain obtained in the section (2-1) (a) in each of feed of a chick (for SD broiler in early stage, manufactured by Nippon Formula Feed Mfg Co., Ltd., a feed supplemented with no antibacterial substance) at a concentration of 100 ppm of the total mass of the feed were used in Examples 42 to 45, respectively. Chicks of the respective groups, each group consisting of 12 chicks hatched from hatching eggs derived from broiler chickens (name: Chunky), were allowed to ingest the feeds of Examples 42 to 45 for 14 days. On the other hand, a feed obtained by mixing the pulverized solid culture of AOK 1006s strain in the feed at a concentration of 100 ppm was defined as Comparative Example 16. A feed obtained by mixing lactose instead of a Koji mold culture at a concentration of 100 ppm was defined as control and used in the test in the same way as above. Each 7-day-old chick was orally challenged by *Clostridium perfringens* (CP) in an amount of 1.1 × 10⁹ CFU. A CP strain isolated from the cecal content of a chicken that had died at a farm of a poultry producer in Gunma-ken was used for the challenge. From 14-day-old chicks, cecal contents were collected, and feces were collected by wiping the cloacae with swabs.

The CP viable cells in the cecal contents were counted by the following method to calculate infection indices and defense indices.
1 g of each cecal content was diluted 10-fold with sterilized phosphate buffered saline, and the solution was sufficiently mixed to prepare a sample stock solution. Subsequently, the sample stock solution was serially diluted 10-fold with sterilized physiological saline to prepare serially-diluted solutions. The sample stock solution and serially-diluted solutions were separately smeared on a medium for Clostridium measurement (manufactured by Nissui Pharmaceutical Co., Ltd.) in an amount of 0.1 ml and anaerobically cultured at 35°C for 24 hours using Anaero Pack Kenki, and the number of black colonies grown on each plate medium was determined. Moreover, bacteria were collected from the colonies and inoculated to egg yolk-supplemented CW agar medium (manufactured by Nissui Pharmaceutical Co., Ltd.), and the media were aerobically and anaerobically cultured at 35°C for 24 to 48 hours to confirm their properties.
The number of CP viable cells per g of a cecal content was calculated by multiplying the number of colonies confirmed to be CP by a dilution rate of a diluted solution. Based on the results, infection indices and defense indices were calculated as the above.

For the feces collected from the cloacae, qualitative culture was performed for each individual by the following method to confirm the properties of CP. That is, the feces attached to swabs were suspended in 10 ml of sterilized phosphate buffered saline to prepare sample stock solutions, and the sample stock solutions was separately smeared on a medium for Clostridium measurement (manufactured by Nissui Pharmaceutical Co., Ltd.) in an amount of 0.1 ml and anaeroically cultured at 35°C for 24 hours, and the presence or absence of formation of black colony grown on each plate medium was estimated. Moreover, bacteria were collected from the colonies and inoculated to an egg yolk-supplemented CW agar medium (manufactured by Nissui Pharmaceutical Co., Ltd.) and the media were aerobically and anaerobically cultured at 35°C for 24 to 48 hours to confirm their properties.
The results are shown in Table 18.

**[Table 18]**

| | Number of viable cells in cecal content | | Number of individuals whose feces collected from cloacae contain CP Number of study chicks (12) |
|---|---|---|---|
| | Infection index | Defense index | |
| Example 42 | 2.9 | 2.2 | 7 |
| Example 43 | 5.3 | 1.3 | 9 |
| Example 44 | 5.8 | 1.2 | 9 |
| Example 45 | 5.5 | 1.3 | 10 |
| Comparative Example 16 | 7.2 | 1.0 | 9 |
| Control | 7.1 | - | 10 |

In the cases of the chicks administered with the feeds containing AOK 210 strain, AOK 43 strain, AOK N4586 strain, and AOK B650 strain of Examples 42 to 45, the concentrations of CP viable cells in the cecal contents were extremely low, and the CP infection indices were extremely low. In Example 42, the defense index was particularly high. In the case of chicks administered with the feed containing AOK 1006s of Comparative Example 16, the number of CP viable cells was almost the same as that of control. The results revealed that the cultures of the present invention containing acidic enzymes produced by *Aspergillus sojae, Aspergillus tamarii, Aspergillus foetidus,* and *Aspergillus niger* had effects of preventing infections caused by CP.

### (6-2) Challenge study of Clostridium perfrigens to chick

A feed obtained by mixing the pulverized solid culture of IK-05074 strain obtained in the section (2-2) (a) in a feed at a concentration of 100 ppm was used in Example 46, a feed obtained by mixing the pulverized solid culture of AOK 1006s strain in a feed at a concentration of 100 ppm was used in Comparative Example 17, and a feed obtained by mixing lactose instead of a Koji mold culture in a feed at a concentration of 100 ppm was defined as control.
A CP challenge study was performed using these feeds to breed chicks. The study was performed in the same way as above except that each chick was orally challenged by CP in an amount of 1.5 × 10⁹ CFU.
The results are shown in Table 19.

**[Table 19]**

| | Number of viable cells in cecal content | | Number of individuals whose feces collected from cloacae contain CP Number of test chicks (12) |
|---|---|---|---|
| | Infection index | Defense index | |
| Example 46 | 2.5 | 2.8 | 6 |
| Comparative Example 17 | 6.5 | 1.1 | 12 |
| Control | 6.9 | - | 12 |

In the cases of the chicks administered with the feed containing IK-05074 strain of Example 46, the concentration of CP viable cells in the cecal content was extremely low, and the CP infection index was extremely low. In the case of chicks administered with the feed containing AOK 1006s of Comparative Example 17, the number of CP viable cells was almost the same as that of control. The results revealed that the culture of the present invention containing an acidic enzyme produced by *Aspergillus oryzae* had an effect of preventing an infection caused by CP.

### (7-1) Challenge study of Escherichia coli to calf

One-week-old male calves (Holstein) of the respective groups, each group consisting of 8 calves, were raised. Feeds obtained by mixing the pulverized solid cultures of AOK 210 strain, AOK 43 strain, AOK N4586 strain, and AOK B650 strain obtained in the section (2-1) (a) in a mixed feed for calf (Miracle Mate, manufactured by Scientific Feed Laboratory Co., Ltd.) at a concentration of 100 ppm based on the total mass of the feed were used in Examples 47 to 50, respectively. The mixed feeds for calves were given to the calves turned 4-week-old. On the other hand, a feed obtained by mixing the pulverized solid culture of AOK 1006s strain in the feed at a concentration of 100 ppm was used in Comparative Example 18. A feed obtained by mixing lactose instead of a Koji mold culture in the feed at a concentration of 100 ppm was defined as control and used in the study in the same way as above. Each 2-week-old calf was orally challenged by *Escherichia coli* (EC) in an amount of 1.2 × 10⁶ CFU. The calves were raised until the calves turned 4-week-old, and death rates of the calves of the respective groups were calculated.

Meanwhile, small intestine contents were collected, and the numbers of EC viable cells in the small intestine contents were determined by the following method.
1 g of each small intestine content was diluted 10-fold with sterilized phosphate buffered saline, and the solution was sufficiently mixed to prepare a sample stock solution. Subsequently, the sample stock solution was serially diluted 10-fold with sterilized physiological saline to prepare serially-diluted solutions. The sample stock solution and serially-diluted solutions were separately smeared on Chromocult coliform agar "Merk" in an amount of 0.1 ml and cultured at 37°C for 24 hours, and the number of typical EC colonies grown on each plate medium was determined. The number of EC viable cells per g of a small intestine content was calculated by multiplying the number of colonies confirmed to be EC by a dilution rate of a diluted solution. Based on the results, infection indices and defense indices were calculated in the same way as above.
The results are shown in Table 20.

**[Table 20]**

| | Death rate 14 days after EC infection (%) | Number of viable cells in small intestine content | |
|---|---|---|---|
| | | Infection index | Defense index |
| Example 47 | 0 | 4.3 | 1.9 |
| Example 48 | 0 | 5.3 | 1.5 |
| Example 49 | 0 | 5.7 | 1.4 |
| Example 50 | 0 | 5.5 | 1.5 |
| Comparative Example 18 | 13 | 7.7 | 1.0 |
| Control | 25 | 8.0 | - |

In the cases of the calves administered with the feeds containing AOK 210 strain, AOK 43 strain, AOK N4586 strain, and AOK B650 strain of Examples 47 to 50, the death rates were 0%. In addition, the EC infection indices in the small intestine contents were low. In Example 47, the defense index was particularly high. In the case of the calves administered with the feed containing AOK 1006s strain of Comparative Example 18, the death rate was 13%. Meanwhile, the number of EC viable cells was almost the same as that of control. The results revealed that the cultures containing acidic enzymes produced by *Aspergillus sojae, Aspergillus tamarii, Aspergillus foetidus,* and *Aspergillus niger* have effects of preventing infections caused by EC.

### (7-2) Challenge study of Escherichia coli to calf

A mixed feed for calf obtained by mixing the pulverized solid culture of IK-05074 strain in a feed at a concentration of 100 ppm was used in Example 51, a feed obtained by mixing the pulverized solid culture of AOK 1006s strain in a feed at a concentration of 100 ppm was used in Comparative Example 19, and a feed obtained by mixing lactose instead of a Koji mold culture in a feed at a concentration of 100 ppm was defined as control. An EC challenge study was performed using the feeds to breed calves. The study was performed in the same way as above except that each calf was orally challenged by EC in an amount of 1.5 × 10⁶ CFU.
The results are shown in Table 21.

**[Table 21]**

| | Death rate 14 days after EC infection (%) | Number of viable cells in small intestine content | |
|---|---|---|---|
| | | Infection index | Defense index |
| Example 51 | 0 | 4.6 | 1.8 |
| Comparative Example 19 | 13 | 7.5 | 1.1 |
| Control | 38 | 8.3 | - |

In the case of the calves administered with the feed containing IK-05074 strain of Example 51, the death rate was 0%. In addition, the EC infection index in the small intestine contents was low. In the case of the calves administered with the feed containing AOK 1006s strain of Comparative Example 19, the death rate was 13%. Meanwhile, the number of EC viable cells was almost the same as that of control. The results revealed that the culture containing an acidic enzyme produced by *Aspergillus oryzae* has an effect of preventing an infection caused by EC.

### (8-1) Challenge study of edema disease pathogen to piglet

Feeds obtained by mixing the pulverized solid cultures of AOK 210 strain, AOK 43 strain, AOK N4586 strain, and AOK B650 strain obtained in the section (2-1) (a) in a feed for piglets (artificial milk for SD piglet in early stage, manufactured by Nippon Formula Feed Mfg Co., Ltd., a feed supplemented with no antibacterial substance) at a concentration of 100 ppm based on the total mass of the feed were used in Examples 52 to 55, respectively. 35-day-old piglets (Large Yorkshire) of the respective groups, each group consisting of 30 piglets, were allowed to ingest the feeds for 19 days. On the other hand, a feed obtained by mixing the pulverized solid culture of AOK 1006s strain in the feed at a concentration of 100 ppm was used in Comparative Example 20. A feed obtained by mixing lactose instead of a Koji mold culture in the feed at a concentration of 100 ppm was defined as control and used in the study in the same way as above. Each 40-day-old piglet was orally challenged by an edema disease pathogen (*Escherichia coli*) in an amount of 2.3 × 10⁵ CFU. The piglets were raised until the piglets turned 54-day-old, and death rates of the piglets of the respective groups were calculated.
Meanwhile, in the same way as above, the small intestine contents were collected, and the numbers of EC viable cells in the small intestine contents were counted to calculate infection indices and defense indices.
The results are shown in Table 22.

**[Table 22]**

| | Death rate 14 days after edema disease infection (%) | Number of viable cells in small intestine content | |
|---|---|---|---|
| | | Infection index | Defense index |
| Example 52 | 23 | 4.2 | 2.1 |
| Example 53 | 30 | 5.5 | 1.6 |
| Example 54 | 33 | 5.3 | 1.7 |
| Example 55 | 30 | 5.6 | 1.6 |
| Comparative Example 20 | 53 | 7.5 | 1.2 |
| Control | 57 | 8.8 | - |

In the cases of the piglets administered with the feeds containing AOK 210 strain, AOK 43 strain, AOK N4586 strain, and AOK B650 strain of Examples 52 to 55, the death rates were about 20 to 30% and were lower than the death rate of the group administered with the feed of Comparative Example 20. Meanwhile, the EC infection indices in the small intestine contents were low, and the defense index of Example 52 was particularly high. In addition, the number of viable cells of the edema disease pathogen in Comparative Example 20 was almost the same as that of control. The results revealed that the cultures of the present invention containing acidic enzymes produced by *Aspergillus sojae, Aspergillus tamarii, Aspergillus foetidus,* and *Aspergillus niger* have effects of preventing edema disease.

### (8-2) Challenge study of edema disease pathogen to piglet

A feed for piglet obtained by mixing the pulverized solid culture of IK-05074 strain obtained in the section (2-2) (a) in a feed at a concentration of 100 ppm was used in Example 56, a feed obtained by mixing the pulverized solid culture of AOK 1006s strain in a feed at a concentration of 100 ppm was defined as Comparative Example 21, and a feed obtained by mixing lactose instead of a Koji mold culture in a feed at a concentration of 100 ppm was defined as control. An edema disease pathogen challenge study was performed using the feeds to breed piglets. The study was performed in the same way as above except that each piglet was orally challenged by the edema disease pathogen in an amount of 1.8 × 10⁵ CFU.
The results are shown in Table 23.

**[Table 23]**

| | Death rate 14 days after edema disease infection (%) | Number of viable cells in small intestine content | |
|---|---|---|---|
| | | Infection index | Defense index |
| Example 56 | 20 | 4.0 | 2.1 |
| Comparative Example 21 | 50 | 7.6 | 1.1 |
| Control | 50 | 8.3 | - |

In the cases of the piglets administered with the feeds containing IK-05074 strain of Examples 56, the death rates were about 20% and were lower than the death rate of the group administered with the feed of Comparative Example 21. Meanwhile, the EC infection indices in the small intestine contents were low. In addition, the number of viable cells of the edema disease pathogen in Comparative Example 21 was almost the same as that of control. The results revealed that the cultures of the present invention containing an acidic enzyme produced by *Aspergillus oryzae* has effect of preventing edema disease.

### (9-1) Coccidium-prevention test

### (a) Eimeria tenella-prevention test

Feces of chickens that had been naturally infected with *Eimeria tenella* were collected, and oocysts were separated under a stereomicroscope and washed with physiological saline. To a dish with a diameter of 9 cm was added 5 ml of physiological saline, and the washed oocysts were added thereto at about 4, 000 oocysts/ml. The pulverized solid cultures of AOK 210 strain, AOK 43 strain, AOK N4586 strain, and AOK B650 strain obtained in the section (2-1) (a) were separately added to dishes in an amount of 50 mg per dish, and the resultant products were used in Examples 57 to 60, respectively. A dish containing 50 mg of the pulverized solid culture of AOK 1006s was defined as Comparative Example 22, and a dish containing no Koji mold culture was defined as control. Those dishes were shaken at 37°C (150 rpm). 7 days later, a stereomicroscope was used to determine the numbers of oocysts and to observe conditions of deformed and dissolved cell walls, and reduction rates of oocysts and rates of dissolved and denatured oocysts.
The results are shown in Table 24.

**[Table 24]**

| | Number of oocysts | | Reduction rate (%) | Rate of dissolved and denatured ¹⁾ (%) |
|---|---|---|---|---|
| | Day 0 | Day 7 | | |
| Example 57 | 4,000 | 400 | 90 | 25 |
| Example 58 | 4,500 | 800 | 82 | 12 |
| Example 59 | 4,400 | 900 | 80 | 11 |
| Example 60 | 4,100 | 900 | 78 | 11 |
| Comparative Example 22 | 4,200 | 3,800 | 10 | 3 |
| Control | 4,400 | 4,400 | 0 | 0 |

| | | | | |
|---|---|---|---|---|
| 1) Rate based on oocysts that remained on day 7 | | | | |

As shown in Examples 57 to 60, in the cases of the solid cultures of AOK 210 strain, AOK 43 strain, AOK N4586 strain, and AOK B650 strain, the numbers of oocysts of *E. tenella* were obviously reduced compared with the solid culture of AOK 1006s shown in Comparative Example 22, and the fungi were found to have higher activity to dissolve and denature oocysts. In particular, in the case where oocysts of *E. tenella* were treated with AOK 210 strain, the reduction rate and rate of dissolved and denatured oocysts were extremely high.

### (b) Eimeria zuernii-prevention test

Diarrheal feces of cows that had been naturally infected with *Eimeria zuernii* were collected, and oocysts were separated under a stereomicroscope and washed with physiological saline. To a dish with a diameter of 9 cm was added 5 ml of physiological saline, and the washed oocysts were added thereto at about 2, 000 oocysts/ml. The pulverized solid cultures of AOK 210 strain, AOK 43 strain, AOK N4586 strain, and AOK B650 strain obtained in the section (2-1) (a) were separately added to dishes in an amount of 50 mg per dish, and the resultant products were used in Examples 61 to 64, respectively. A dish containing 50 mg of the pulverized solid culture of AOK 1006s was defined as Comparative Example 23, and a dish containing no Koji mold culture was defined as control. Those dishes were shaken at 37°C (150 rpm). 7 days later, a stereomicroscope was used to determine the numbers of oocysts and to observe conditions of deformed and dissolved cell walls, and reduction rates of oocysts and rates of dissolved and denatured oocysts.
The results are shown in Table 25.

**[Table 25]**

| | Number of oocysts | | Reduction rate (%) | Rate of dissolved and denatured ¹⁾ (%) |
|---|---|---|---|---|
| | Day 0 | Day 7 | | |
| Example 61 | 2,200 | 600 | 73 | 20 |
| Example 62 | 2,200 | 1,100 | 50 | 9 |
| Example 63 | 2,000 | 1,100 | 45 | 8 |
| Example 64 | 2,300 | 1,200 | 48 | 8 |
| Comparative Example 23 | 2,000 | 1,800 | 10 | 3 |
| Control | 2,300 | 2,300 | 0 | 0 |

| | | | | |
|---|---|---|---|---|
| 1) Rate based on oocysts that remained on day 7 | | | | |

As shown in Examples 61 to 64, in the cases of the solid cultures of AOK 210 strain, AOK 43 strain, AOK N4586 strain, and AOK B650 strain, the numbers of oocysts of *E. zuernii* were obviously reduced compared with the solid culture of AOK 1006s shown in Comparative Example 23, and the fungi were found to have higher activity to dissolve and denature oocysts. In particular, in the case where oocysts of *E. zuernii* were treated with AOK 210 strain, the reduction rate and rate of dissolved and denatured oocysts were extremely high.

### (9-2) Coccidium-prevention test

### (a) Eimeria tenella-prevention test

A dish containing the pulverized solid culture of IK-05074 strain obtained in the section (2-2) (a) was defined as Example 65, and an *Eimeria tenella*-prevention test was performed in the same way as above. Meanwhile, a dish containing the solid culture of AOK 1006s strain was used in Comparative Example 24 and used in the test in the same way as above.
The results are shown in Table 26.

**[Table 26]**

| | Number of oocysts | | Reduction rate (%) | Rate of dissolved and denatured ¹⁾ (%) |
|---|---|---|---|---|
| | Day 0 | Day 7 | | |
| Example 65 | 4,200 | 380 | 91 | 29 |
| Comparative Example 24 | 4,000 | 3,500 | 13 | 4 |
| Control | 4,100 | 4,100 | 0 | 0 |

| | | | | |
|---|---|---|---|---|
| 1) Rate based on oocysts that remained on day 7 | | | | |

As shown in Example 65, the solid culture of IK-05074 strain was found to have an ability to reduce the number of oocysts of *E. tenella* and have higher activity to dissolve and denature oocysts compared with the solid culture of AOK 1006s strain shown in Comparative Example 24.

### (b) Eimeria zuernii-prevention test

A dish containing the pulverized solid culture of IK-05074 strain obtained in the section (2-2) (a) was defined as Example 66, and an *Eimeria zuernii*-prevention test was performed in the same way as above. Meanwhile, a dish containing the solid culture of AOK 1006s was defined as Comparative Example 25 and used in the test in the same way as above.
The results are shown in Table 27.

**[Table 27]**

| | Number of oocysts | | Reduction rate (%) | Rate of dissolved and denatured ¹⁾ (%) |
|---|---|---|---|---|
| | Day 0 | Day 7 | | |
| Example 66 | 2,000 | 500 | 75 | 31 |
| Comparative Example 25 | 2,200 | 1,800 | 18 | 3 |
| Control | 2,000 | 2,000 | 0 | 0 |

| | | | | |
|---|---|---|---|---|
| 1) Rate based on oocysts that remained on day 7 | | | | |

As shown in Example 66, the solid culture of IK-05074 strain was found to have an ability to reduce the number of oocysts of *E. zuernii* and have higher activity to dissolve and denature oocysts compared with the solid culture of AOK 1006s strain shown in Comparative Example 25.

### Industrial Applicability

If the additive for animal feed of the present invention which comprises the culture containing an *Aspergillus* fungus and an acidic enzyme produced by the fungus is mixed in a feed and administered to an animal, absorption of nutrients is promoted to elevate the feed efficiency. Meanwhile, the concentration of *Aspergillus* fungus is increased in the intestine of the animal, resulting in improvement of the balance of enterobacterial flora. Moreover, proliferation of a pathogen and a coccidium is suppressed to prevent/improve intestinal infections. The feed of the present invention can be suitably used in breeding of livestock animals such as a chicken, pig, and cow.

## Claims

1. An additive for animal feed, comprising a culture containing at least one fungus selected from *Aspergillus sojae, Aspergillus tamarii, Aspergillus foetidus, Aspergillus niger,* and *Aspergillus oryzae*, and an acidic enzyme produced by the fungus.

2. The additive for animal feed according to Claim 1, wherein the fungus is *Aspergillus sojae* and/or *Aspergillus oryzae.*

3. The additive for animal feed according to Claim 1 or 2, wherein the *Aspergillus oryzae* is *Aspergillus oryzae* IK-05074 strain (FERM BP-10622) or a mutant of the *Aspergillus oryzae* IK-05074 strain having the same ability to produce acidic enzyme as the *Aspergillus oryzae* IK-05074 strain.

4. The additive for animal feed according to any one of Claims 1 to 3, wherein the acidic enzyme is an acid amylase.

5. The additive for animal feed according to any one of Claims 1 to 4, wherein the fungus has antibacterial activity against a pathogen that causes an intestinal infection of an animal and/or protozoa-killing activity against a coccidium.

6. The additive for animal feed according to any one of Claims 1 to 5, wherein the culture contains a plant-derived nutrient.

7. The additive for animal feed according to Claim 6, wherein the plant-derived nutrient is brown rice.

8. A feed comprising the additive for animal feed according to any one of Claims 1 to 7.

9. A method of manufacturing a feed, comprising:
culturing at least one fungus selected from *Aspergillus sojae, Aspergillus tamarii, Aspergillus foetidus, Aspergillus niger,* and *Aspergillus oryzae* on a solid medium containing a nutrient for proliferation of the fungus; and
adding the resultant culture to a feed.
